(19) Europäisches Patentamt
European Patent Office
Office européen des brevets

(11) **EP 4 786 441 A1**

(12) **EUROPEAN PATENT APPLICATION**
published in accordance with Art. 153(4) EPC

(43) Date of publication:
**05.08.2026 Bulletin 2026/32**

(21) Application number: **24872269.6**

(22) Date of filing: **25.09.2024**

(51) International Patent Classification (IPC):
***C04B 35/488*** (2006.01)

(52) Cooperative Patent Classification (CPC):
**A61K 6/818; A61K 6/84; C01G 25/02; C04B 35/488**

(86) International application number:
**PCT/JP2024/034159**

(87) International publication number:
**WO 2025/070483 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 JP 2023165892**

(71) Applicant: **Tosoh Corporation**
**Shunan-shi, Yamaguchi 746-8501 (JP)**

(72) Inventors:
• **FUKIAGE, Taku**
**Shunan-shi Yamaguchi 746-8501 (JP)**
• **TAKESUE, Takamasa**
**Shunan-shi Yamaguchi 746-8501 (JP)**
• **KAWAMURA, Kiyotaka**
**Shunan-shi Yamaguchi 746-8501 (JP)**
• **NAGAYAMA, Hitoshi**
**Shunan-shi Yamaguchi 746-8501 (JP)**

(74) Representative: **Müller-Boré & Partner**
**Patentanwälte PartG mbB**
**Friedenheimer Brücke 21**
**80639 München (DE)**

(54) **ZIRCONIA SINTERED BODY AND POWDER**

(57)     To provide at least one selected from the group consisting of a sintered body, a powder, a green body and a calcined body in which both processability and light transmitting properties are achieved and cerium-derived color is reduced.

A sintered body of zirconia comprises a color reducing agent and cerium as a stabilizing element, in which the sintered body has a cerium content of 0.05 mol% or more and 15 mol% or less and a color reducing agent content of 0.01 mass% or more and less than 1.95 mass%.

EP 4 786 441 A1

**Description**

TECHNICAL FIELD

The present disclosure relates to a sintered body, a powder, a green body and a calcined body of zirconia in which both processability and light transmitting properties are achieved and the color derived from cerium is reduced.

BACKGROUND ART

A sintered body containing zirconia as the matrix (hereinafter may also be referred to as a "zirconia sintered body") is a high-strength, chemically stable material that also has light transmitting properties. Due to these characteristics, such sintered bodies have been increasingly used as structural materials, decorative materials and dental materials in recent years.

Meanwhile, a sintered body of zirconia has high hardness and thus low processability, and tools used for processing wear out rapidly. Thus, when preparing a sintered body of zirconia, processing is usually performed on a green body or a calcined body before sintering and then sintering is performed. However, when processing is performed before sintering, a processing allowance to account for the sintering shrinkage must be incorporated into the design, and processing for fine adjustment becomes necessary after the sintering. This decreases processing efficiency and increases waste. In recent years, demand for high-processability zirconia sintered bodies that can be processed in a sintered body state has grown in various applications including dental material applications in order to improve the processing efficiency and reduce waste in view of the sustainable development goals (hereinafter may also be referred to as the "SDGs").

Meanwhile, zirconia that contains cerium as a stabilizing element has low hardness and high fracture toughness compared with zirconia containing yttrium as a stabilizing element and is thus suitable for sintered body processing. For example,

Patent Document 1 discloses that a zirconia sintered body containing cerium and yttrium as stabilizing elements and prepared by setting the sintering temperature to 1450°C to 1550°C exhibits low hardness and high fracture toughness.

PRIOR ART DOCUMENTS

PATENT DOCUMENTS

Patent Document 1: JP-A-2021-091602

DISCLOSURE OF INVENTION

TECHNICAL PROBLEM

However, zirconia containing cerium as a stabilizing element has yellow color derived from cerium and has a color tone different from typical zirconia sintered bodies. Thus, the applications to which sintered bodies of zirconia containing cerium can be applied are limited due to this color tone.

Although the yellow color derived from cerium can be lightened by adding alumina and pigments, such addition decreases the light transmitting properties of the sintered bodies. Furthermore, although increasing the stabilizing element content improves the light transmitting properties of the sintered bodies of zirconia, improved light transmitting properties intensify the color derived from cerium and decreases the fracture toughness. Thus, cracking and chipping are likely to occur during processing. As such, existing sintered bodies of zirconia containing cerium as a stabilizing element could not have the color derived from cerium reduced while achieving both processability and light transmitting properties.

An object of the present disclosure is to provide at least one selected from the group consisting of a sintered body, a powder, a green body and a calcined body in which both processability and light transmitting properties are achieved and the color derived from cerium is reduced.

SOLUTION TO PROBLEM

The present invention is disclosed by the claims, and the gist of the present disclosure is as follows.

[1] A sintered body of zirconia, the sintered body comprising a color reducing agent and cerium as a stabilizing element, wherein the sintered body has a cerium content of 0.05 mol% or more and 15 mol% or less and a color reducing agent content of 0.01 mass% or more and less than 1.95 mass%.

[2] The sintered body described in [1] above, comprising at least one selected from the group consisting of scandium, yttrium, praseodymium, gadolinium, terbium, erbium, magnesium and calcium as a stabilizing element other than cerium.

[3] The sintered body described in [2] above, wherein a content of the stabilizing element other than cerium is more than 0 mol% and 2.8 mol% or less when the stabilizing element other than cerium is at least one selected from the group consisting of scandium, yttrium, praseodymium, gadolinium, terbium and erbium, and is more than 0 mol% and 9.8 mol% or less when the stabilizing element other than cerium is either or both of magnesium and calcium.

[4] The sintered body described in any one of [1] to [3] above, comprising more than 0 mass% and 5.0 mass% or less of alumina.

[5] The sintered body described in any one of [1] to [4] above, wherein the color reducing agent contains at least one element that has an 8-coordinate ionic radius larger than an ionic radius of a tetravalent cerium ion.

[6] The sintered body described in any one of [1] to [5] above, wherein the color reducing agent contains at least one selected from the group consisting of lanthanum, neodymium, samarium, europium, dysprosium, holmium, thulium, ytterbium and lutetium.

[7] The sintered body described in any one of [1] to [6] above, wherein the sintered body has a color tone (L*a*b*) satisfying formulae (1) and (2) below in L*a*b* color space:

Formula (1) …

$$0.34 \times L^* - 32.5 < a^* < 0.34 \times L^* - 20$$

Formula (2) …

$$-1.3 \times L^* + 70 < b^* < -1.3 \times L^* + 115.$$

[8] The sintered body described in any one of [1] to [7] above, wherein the sintered body has a chroma C* of 12.0 or less.

[9] The sintered body described in any one of [1] to [8] above, wherein the sintered body has a Vickers hardness Hv10 of 600 or more and 1200 or less.

[10] The sintered body described in any one of [1] to [9] above, wherein the sintered body has a total luminous transmittance at a thickness of 1 mm of 20% or more and 70% or less.

[11] A powder of zirconia, the powder comprising a color reducing agent source and a cerium compound as a stabilizing element source, wherein the powder has a cerium content of 0.05 mol% or more and 15 mol% or less and a color reducing agent content of 0.01 mass% or more and less than 1.95 mass%.

[12] The powder described in [11] above, wherein the stabilizing element source includes the cerium compound and a compound of either or both of yttrium and magnesium.

[13] The powder described in [11] or [12] above, wherein the color reducing agent source is a compound containing at least one selected from the group consisting of lanthanum, neodymium, samarium, europium, dysprosium, holmium, thulium, ytterbium and lutetium.

DESCRIPTION OF EMBODIMENTS

A sintered body of the present disclosure will now be described through some exemplary embodiments. Furthermore, the present disclosure encompasses any desired combinations of the features and parameters disclosed in this description and any combinations of upper limits and lower limits of the values disclosed in this description.

A sintered body according to an embodiment is a sintered body of zirconia, comprising a color reducing agent and cerium as a stabilizing element, in which the sintered body has a cerium content of 0.05 mol% or more and 15 mol% or less and a color reducing agent content of 0.01 mass% or more and less than 1.95 mass%.

The sintered body of the present embodiment is a ceramic sintered body and is a sintered body containing zirconia as the matrix (sintered body containing zirconia as the main component), also known as zirconia sintered body.

The sintered body of the present embodiment contains a stabilizing element. The sintered body of the present embodiment contains cerium (Ce) as a stabilizing element, and the main stabilizing element is cerium. Thus, cerium is solidly dissolved in zirconia. Since cerium is the stabilizing element, the sintered body exhibits high processability.

The sintered body of the present embodiment may contain a stabilizing element other than cerium (hereinafter such an element may also be referred to as a "sub-stabilizing element") and preferably contains a sub-stabilizing element. In this manner, the sintering temperature range in which a dense sintered body with high mechanical properties is obtained expands, and, in producing the sintered body of the present embodiment, a sintered body is more stably and easily obtained. The sub-stabilizing element is preferably at least one selected from the group consisting of scandium (Sc), yttrium (Y), praseodymium (Pr), gadolinium (Ga), terbium (Tb), erbium (Er), magnesium (Mg) and calcium (Ca), at least one selected from the group consisting of magnesium, calcium, yttrium, erbium and scandium, at least one of yttrium and magnesium or yttrium.

A particularly preferable combination of the stabilizing elements is a combination of cerium and at least one selected from the group consisting of magnesium, calcium, yttrium, erbium and scandium, a combination of cerium and at least one of yttrium and magnesium or a combination of cerium and yttrium, for example.

The content of the stabilizing element (hereinafter may also be referred to as the "stabilizing element amount") in the sintered body of the present embodiment is the total amount of all stabilizing elements contained in the sintered body and is the amount at which zirconia is partially stabilized. The stabilizing element amount on an oxide basis is preferably 0.05 mol% or more, 1 mol% or more, 2 mol% or more, 3 mol% or more, 6 mol% or more or 7 mol% or more and is preferably 20 mol% or less, 15 mol% or less or 10 mol% or less. The oxide-based stabilizing element amount of the sintered body of the present embodiment is, is, for example, 0.05 mol% or more and 20 mol% or less, and is more preferably 1 mol% or more and 20 mol% or less, 2 mol% or more and 20 mol% or less, 3 mol% or more and 20 mol% or less, 4 mol% or more and 20 mol% or less, 4 mol% or more and 15 mol% or less, 4 mol% or more and 10 mol% or less, 6 mol% or more and 15 mol% or less or 7 mol% or more and 15 mol% or less.

The content of cerium (hereinafter may also be referred to as the "cerium amount") is 0.05 mol% or more and 15 mol% or less. At a cerium amount exceeding 15 mol%, generation of cubic phase is induced, and mechanical properties are degraded. At a cerium amount less than 0.05 mol%, generation of monoclinic phase is induced, and it becomes difficult to obtain a sintered body due to factors such as a tendency to crack during sintering. The cerium amount is preferably 1 mol% or more, 2 mol% or more, 3 mol% or more or 3.5 mol% or more and is preferably 15 mol% or less, 12 mol% or less, 10 mol% or less or 8.5 mol% or less. The cerium amount of the sintered body of the present embodiment is preferably 1 mol% or more and 15 mol% or less, 2 mol% or more and 15 mol% or less, 3 mol% or more and 15 mol% or less, 3.5 mol% or more and 15 mol% or less, 3.5 mol% or more and 12 mol% or less, 3.5 mol% or more and 10 mol% or less or 3.5 mol% or more and 8.5 mol% or less.

When the sintered body of the present embodiment contains a sub-stabilizing element, the lower limit of the content of the sub-stabilizing element is preferably more than 0 mol%, 0.5 mol% or more or 1 mol% or more, and the upper limit is preferably 10 mol% or less, 8 mol% or less or 7 mol% or less. The content of the sub-stabilizing element is preferably more than 0 mol% and 10 mol% or less, 0.5 mol% or more and 8 mol% or less or 1 mol% or more and 7 mol% or less.

When at least one selected from the group consisting of scandium, yttrium, praseodymium, gadolinium, terbium and erbium is contained as a sub-stabilizing element, the total content (when there is only one sub-stabilizing element, the amount of that element contained; hereinafter this total content may also be referred to as the "sub-stabilizing element amount") is preferably more than 0 mol% and 2.8 mol% or less, 0.05 mol% or more and 2.8 mol% or less, 0.1 mol% or more and 2.8 mol% or less, 0.5 mol% or more and 2.8 mol% or less, 0.5 mol% or more and 2.5 mol% or less, 0.5 mol% or more and 2.2 mol% or less, 0.5 mol% or more and 2.0 mol% or less, 0.5 mol% or more and 1.8 mol% or less, 0.5 mol% or more and 1.5 mol% or less or 0.5 mol% or more and 1.1 mol% or less. When the content of these elements is 2.8 mol% or less, the sintered body exhibits hardness suitable for processing.

When either or both of magnesium and calcium are contained as the sub-stabilizing element, the sub-stabilizing element amount is preferably more than 0 mol% and 9.8 mol% or less, 0.05 mol% or more and 9.8 mol% or less, 0.1 mol% or more and 9.8 mol% or less, 0.5 mol% or more and 9.8 mol% or less, 0.5 mol% or more and 9.1 mol% or less, 0.5 mol% or more and 7.1 mol% or less or 0.5 mol% or more and 5.1 mol% or less. In this manner, the sintered body exhibits hardness suitable for processing.

In the present embodiment, the stabilizing element amount may be determined from the ratio (mol%) of the total of the stabilizing elements as oxides to the total of zirconia and the stabilizing elements as oxides. For example, the stabilizing element amount of a sintered body (or a powder) containing zirconia containing cerium and yttrium can be determined by $\{(CeO_2 + Y_2O_3)/(CeO_2 + Y_2O_3 + ZrO_2)\} \times 100$ (mol%) by using $ZrO_2$ as zirconium, $CeO_2$ as cerium and $Y_2O_3$ as yttrium.

In the present embodiment, calculating the amounts of the stabilizing elements as oxides may involve using $CeO_2$ as cerium, $MgO$ as magnesium, $CaO$ as calcium, $Y_2O_3$ as yttrium, $Sc_2O_3$ as scandium, $Gd_2O_3$ as gadolinium, $Er_2O_3$ as erbium, $Pr_6O_{11}$ as praseodymium and $Tb_4O_7$ as terbium.

In the sintered body of the present embodiment, the main stabilizing element is preferably cerium, and the sub-stabilizing element amount is equal to or less than the cerium amount and is preferably less than the cerium amount. The molar ratio [mol/mol] of the sub-stabilizing element to cerium is, for example, less than 0.5, 0.3 or less or 0.2 or less and 0 or more, more than 0 or 0.1 or more. The range of the molar ratio of the sub-stabilizing element to cerium is, for example, 0 or more and less than 0.5 or 0 or more and 0.2 or less, and the range of the molar ratio of the sub-stabilizing element to cerium when the sub-stabilizing element is contained is, for example, more than 0 and less than 0.5, more than 0 and 0.3 or less, more than 0 and 0.2 or less, 0.1 or more and less than 0.5, 0.1 or more and 0.3 or less or 0.1 or more and 0.2 or less.

The stabilizing element is preferably solidly dissolved in zirconia, and the sintered body of the present embodiment is preferably free of undissolved stabilizing elements. The absence of the undissolved stabilizing elements may be confirmed through the absence of the XRD peaks corresponding to the compounds of these stabilizing elements in an XRD pattern.

The sintered body of the present embodiment contains a color reducing agent. The color reducing agent is an element that has a function of reducing the color derived from cerium in the sintered body. The color reducing agent is to contain an element that reduces the color derived from cerium in the sintered body and preferably contains at least one element that has an 8-coordinate ionic radius larger than an ionic radius of tetravalent cerium ion. One of the reasons why such an element functions as a color reducing agent is that such an element segregates in a state that affects the crystal structure of the crystal grains of zirconia constituting the sintered body, thus changing the optical properties of the sintered body and suppressing coloring by cerium. As a specific example of the color reducing agent, at least one selected from the group consisting of lanthanum (La), neodymium (Nd), samarium (Sm), europium (Eu), dysprosium (Dy), holmium (Ho), thulium (Th), ytterbium (Yb) and lutetium (Lu) is more preferably contained, at least one selected from the group consisting of lanthanum, neodymium, samarium, europium, gadolinium, dysprosium and holmium is more preferably contained, at least one selected from the group consisting of lanthanum, neodymium, samarium and dysprosium is yet more preferably contained, either or both of lanthanum and neodymium are still more preferably contained, and lanthanum is particularly preferably contained.

In the present embodiment, the ionic radius is the ionic radius defined in Shannon (Shannon et al., Acta A32 (1976) 751). For example, the ionic radius of the tetravalent ion of cerium ($Ce^{4+}$) in the 8-coordinated state is 111 pm, and the 8-coordinate ionic radius of an element preferable as the reducing agent is 130 pm when the element is lanthanum, 125 pm when the element is neodymium, 122 pm when the element is samarium, 121 pm when the element is europium, 117 pm when the element is dysprosium, 116 pm when the element is holmium, 113 pm when the element is thulium, 113 pm when the element is ytterbium and 112 pm when the element is lutetium.

When the sintered body contains a color reducing agent, the color derived from cerium is reduced even at a small dose. In this manner, the sintered body of the present embodiment exhibits a color tone comparable to the white color tone, that is, the color tone inherent to zirconia, while maintaining the strength and the hardness properties inherent to the sintered body containing cerium as the main stabilizing element.

The content of the color reducing agent (hereinafter may also be referred to as the "color reducing agent amount" and when the color reducing agent is lanthanum or the like, the amount is referred to as the "lanthanum amount" or the like) is preferably 0.01 mass% or more and less than 1.95 mass%, 0.01 mass% or more and 10 mass% or less, 0.01 mass% or more and 8 mass% or less, 0.01 mass% or more and 6 mass% or less, 0.01 mass% or more and 4 mass% or less, 0.01 mass% or more and 2 mass% or less or 0.01 mass% or more and 1.5 mass% or less. When the amount of the color reducing agent exceeds 1.95 mass%, the transmittance of the sintered body decreases, and when the amount of the color reducing agent is less than 0.01 mass%, it becomes difficult to reduce the color derived from cerium. The content of the color reducing agent can be determined as the ratio of the total mass of the color reducing agent on an oxide basis to the mass of the sintered body of the present embodiment, that is, the total of the masses of metal elements as oxides contained in the sintered body. The calculation of the amount of the color reducing agent as oxides involves using $La_2O_3$ as lanthanum, $Nd_2O_3$ as neodymium, $Sm_2O_3$ as samarium, $Eu_2O_3$ as europium, $Dy_2O_3$ as dysprosium, $Ho_2O_3$ as holmium, $Tm_2O_3$ as thulium, $Yb_2O_3$ as ytterbium and $Lu_2O_3$ as lutetium.

The sintered body of this embodiment may contain additive components in addition to the color reducing agent.

The sintered body of the present embodiment may contain alumina ($Al_2O_3$) as an additive component, and when alumina is contained, the content of alumina as oxide is preferably more than 0 mass% and 5.0 mass% or less and is more preferably more than 0 mass% and 3.0 mass% or less, more than 0 mass% and 2.0 mass% or less or more than 0 mass% and 1.0 mass% or less. As long as the content of alumina is 5.0 mass% or less, the light transmitting properties of the sintered body are rarely degraded. The sintered body of the present embodiment may be free of additive components; for example, the additive component amount may be 0 mass% or more and 1.0 mass% or less, 0 mass% or more and 0.1 mass% or less or 0 mass% or more and 0.03 mass% or less.

The sintered body of the present embodiment may contain a pigment component as an additive component. The pigment component is more preferably at least one selected from the group consisting of titanium (Ti), vanadium (V), chromium (Cr), manganese (Mn), iron (Fe), cobalt (Co), nickel (Ni), copper (Cu) and zinc (Zn) and yet more preferably at least one selected from the group consisting of chromium, iron, cobalt, manganese and nickel.

The content of the pigment component is preferably more than 0 mass% and 5 mass% or less, more than 0 mass% and 3 mass% or less, more than 0 mass% and 1.5 mass% or less, more than 0 mass% and 1 mass% or less, more than 0 mass% and 0.8 mass% or less or more than 0 mass% and 0.6 mass% or less. Calculation of the content as oxide may involve using $TiO_2$ as titanium, $V_2O_5$ as vanadium, $Cr_2O_3$ as chromium, $MnO_2$ as manganese, $Fe_2O_3$ as iron, $CoO$ as cobalt, $NiO$ as nickel, $CuO$ as copper and $ZnO$ as zinc.

The sintered body of the present embodiment may contain at least one selected from the group consisting of silica ($SiO_2$), gallium oxide ($Ga_2O_3$) and germanium oxide ($GeO_2$). When silica etc., are contained, a sintered body with further improved mechanical strength can be easily obtained.

The sintered body of the present embodiment may contain inevitable impurities such as hafnia ($HfO_2$) but is

preferably free of anything that are not stabilizing elements, zirconia, the color reducing agent, alumina or pigment components that are used as necessary or inevitable impurities. In the present embodiment, the content of each component in the sintered body may be calculated by considering hafnia as zirconia ($ZrO_2$).

For example, when the sintered body of the present embodiment is a sintered body of zirconia containing, as additive components, a complex oxide represented by $ABO_3$ or $AB_2O_4$, lanthanum oxide ($La_2O_3$), alumina and silica, and, as stabilizing elements, cerium and yttrium, the content of each component may be determined as follows. In the equations below, the mass of $HfO_2$ is considered to be included in the term $ZrO_2$.

Pigment component amount [mass%] = $\{(ABO_3 + AB_2O_4)/(Ce_2O + Y_2O_3 + ZrO_2 + La_2O_3 + Al_2O_3 + SiO_2 + ABO_3 + AB_2O_4)\} \times 100$

Alumina amount [mass%]= $\{Al_2O_3/(Ce_2O + Y_2O_3 + ZrO_2 + La_2O_3 + Al_2O_3 + SiO_2 + ABO_3 + AB_2O_4)\} \times 100$

Silica amount [mass%] = $\{SiO_2/(Ce_2O + Y_2O_3 + ZrO_2 + La_2O_3 + Al_2O_3 + SiO_2 + ABO_3 + AB_2O_4)\} \times 100$

Additive component amount [mass%] = $\{(ABO_3 + AB_2O_4 + Al_2O_3 + SiO_2)/(Ce_2O + Y_2O_3 + ZrO_2 + La_2O_3 + Al_2O_3 + SiO_2 + ABO_3 + AB_2O_4)\} \times 100$

Stabilizing element amount [mol%] = $\{(Ce_2O + Y_2O_3)/(Ce_2O + Y_2O_3 + ZrO_2)\} \times 100$

Cerium amount [mol%] = $\{(Ce_2O)/(Ce_2O + Y_2O_3 + ZrO_2)\} \times 100$

Yttrium amount [mol%] = $\{(Y_2O_3)/(Ce_2O + Y_2O_3 + ZrO_2)\} \times 100$

The applications of the sintered body of the present embodiment are not particularly limited and may be any applications to which sintered bodies of zirconia can be applied. Since the sintered body has high aesthetic merit and strength, the sintered body is suitable for dental materials, decorative articles, covers of accessories such as watches and casings and exterior members of portable electronic devices such as cellular phones. Furthermore, since the hardness is low and the processability is excellent, the material is particularly suitable for sintered body processing and is effective for improving the processing efficiency and reducing waste, thus contributing to sustainable use of resources as specified by SDGs.

In particular, since the sintered body of the present embodiment has a color tone very similar to natural teeth due to the color reducing agent that reduces the color derived from cerium and has excellent processability, toughness and light transmitting properties, the sintered body is used in dental applications, in particular, mill blanks for denture materials, etc., and orthodontic brackets.

The sintered body of the present embodiment preferably has a white color tone by having the yellow coloration derived from cerium reduced by using the color reducing agent. More preferably, the color tone (L*, a*, b*) of a 1-mm thick sintered body in the L*a*b* color space measured with a D65 light source satisfies formulae (1) and (2) below.

Formula (1) …

$$0.34 \times L^* - 32.5 < a^* < 0.34 \times L^* - 20$$

Formula (2) …

$$-1.3 \times L^* + 70 < b^* < -1.3 \times L^* + 115$$

When a* is a value smaller than the upper limit of formula (1), the red color in the sintered body is suppressed, and when a* is a value greater than the lower limit of formula (1), the green color in the sintered body is suppressed. When a* satisfies formula (1), a color tone similar to natural teeth is exhibited. When b* is less than the upper limit of formula (2), the yellow color in the sintered body is suppressed, and when b* is beyond the range of formula (2), the blue color in the sintered body is suppressed. When b* satisfies formula (2), a color tone similar to natural teeth is exhibited. When formulae (1) and (2) are simultaneously satisfied, a sintered body that has prominently high aesthetic merit as the dental material is obtained.

In addition, the value of a* more preferably satisfies formula (1)* below so that aesthetic merit as the dental material is further improved.

$$\text{Formula (1)*}\ldots$$

$$0.34 \times L^* - 32.5 < a^* < 0.34 \times L^* - 18$$

In addition, the value of b* more preferably satisfies formula (2)* below so that aesthetic merit as the dental material is further improved.

$$\text{Formula (2)*}\ldots$$

$$-1.3 \times L^* + 90 < b^* < -1.3 \times L^* + 115$$

The value of lightness L* of the sintered body of the present embodiment is 55 or more, 60 or more, 70 or more or 75 or more and 90 or less, 88 or less or 80 or less. The lightness L* is preferably 60 or more and 88 or less, 65 or more and 85 or less, 70 or more and 85 or less, 75 or more and 85 or less or 75 or more and 80 or less. When the value of L* is 90 or less, the reflectance decreases, and this is preferable as the dental material. When the value of L* is 55 or more, the sintered body exhibits a lightened black color and exhibits a color tone close to the natural teeth, and thus the aesthetic merit as the dental material is improved.

The sintered body of the present embodiment preferably has a chroma C* of 12.0 or less, 11.0 or less or 10.0 or less. The chroma C* is an index of vividness of the color, and the higher the chroma, the more intense the shade. The sintered body of the present embodiment exhibits a color tone that can be visualized as a white color tone when the chroma C* is 12.0 or less. When the sintered body of the present embodiment is achromatic, that is, white that does not have any shade, the chroma C* is 0. Thus, the sintered body of the present embodiment has, for example, a chroma C* of 0 or more, 2.0 or more, 3.0 or more or 4.0 or more or has a chroma C* of 0 more and 12.0 or less, 0 or more and 11.0 or less, 2.0 or more and 11.0 or less or 4.0 or more and 10.0 or less.

The chroma C* is a value determined from the following equation and the hue a* and b*.

$$C^* = \{(a^*)^2 + (b^*)^2\}^{0.5}$$

The sintered body of the present embodiment preferably satisfies both the aforementioned chroma C* and lightness L*.

In the present embodiment, the color tone of the sintered body is measured by a method compliant with JIS Z 8722. An example of a specific measurement method involves using a common spectrophotometric colorimeter (instrument name: CM-700d produced by KONICA MINOLTA JAPAN, INC.) in a black background measurement mode using a black back plate. The measurement conditions are as follows.

Light source: D-65 light source
Viewing angle: 10°
Measurement system: SCI

A disk-shaped sintered body 20 mm diameter $\times$ 1.0 $\pm$ 0.1 mm thickness having a surface roughness Ra $\leq$ 0.02 $\mu$m on both surfaces is used as a sintered body sample for measurement. The color tone evaluation effective area is a diameter of 10 mm.

The sintered body of the present embodiment preferably has light transmitting properties, and more preferably has a total luminous transmittance of 20% or more and 70% or less, 25% or more and 65% or less, 30% or more and 62% or less, 31% or more and 60% or less, 32% or more and 58% or less, 33% or more and 55% or less, 34% or more and 52% or less or 35% or more and 51% or less as measured by the method described below. When the total luminous transmittance of the sintered body is within this range, the sintered body can exhibit appropriate light transmitting properties and the aesthetic merit can be improved especially for the use as dental materials.

In the present embodiment, the total luminous transmittance is measured as a ratio [%] of transmitted light (total of direct and diffuse light) relative to incident light measured in accordance with JIS K 7361-1 from a measurement sample having a sample thickness of 1.0 $\pm$ 0.1 mm. A disk-shaped sintered body having a sample thickness of 1.0 $\pm$ 0.1 mm and a surface roughness Ra $\leq$ 0.02 $\mu$m on both surfaces may be used as the measurement sample, and a haze meter (for example, haze meter NDH4000 produced by NIPPON DENSHOKU INDUSTRIES Co., Ltd.) equipped with a D65 light source as the light source may be used as the measurement instrument. In other words, the total luminous transmittance in the present embodiment is the total luminous transmittance for the D65 light source at a sample thickness of $1.0 \pm 0.1$ mm.

The density of the sintered body of the present embodiment is preferably high, and, for example, the measured density measured by the following method is 5.50 $g/cm^3$ or more, 5.60 $g/cm^3$ or more, 5.70 $g/cm^3$ or more or 5.80 $g/cm^3$ or more and less than 6.50 $g/cm^3$, 6.40 $g/cm^3$ or less, 6.30 $g/cm^3$ or less or 6.20 $g/cm^3$ or less, or is 5.50 $g/cm^3$ or more and less than 6.50 $g/cm^3$ or 5.80 $g/cm^3$ or more and 6.20 $g/cm^3$ or less. When the density of the sintered body is within this range, the monoclinic phase fraction of the sintered body is unlikely to be high, and the mechanical strength tends to improve.

In the present embodiment, the measured density can be calculated by the method compliant with JIS R 1634 (also known as Archimedes' method) and is a value calculated as the mass determined by the mass measurement relative to the volume determined by the Archimedes' method.

The crystal phase of zirconia of the sintered body of the present embodiment preferably contains at least a tetragonal phase and more preferably contains a tetragonal phase as the main phase. The crystal phase of zirconia may be composed of tetragonal and monoclinic phases or tetragonal, monoclinic and cubic phases.

The shape of the sintered body of the present embodiment is, for example, at least one selected from the group consisting of a spherical shape, an approximately spherical shape, an elliptical shape, a disk shape, a cylindrical shape, a cubic shape, a rectangular parallelepiped shape, a polyhedral shape and an approximately polyhedral shape. Furthermore, the shape may be any shape that attains the intended goals, such as various applications.

A method for producing a sintered body according to the present embodiment will now be described.

The method for producing a sintered body of the present embodiment may be any as long as the aforementioned sintered body is obtained, and an example thereof is a production method that includes a step of sintering at least one of a green body (compact) composed of a raw material composition containing a stabilizing element source, zirconia and a color reducing agent source and a calcined body obtained by calcining this green body (hereinafter this step may also be referred to as the "sintering step").

The green body (compact) subjected to the sintering step is a green body composed of a raw material composition containing a stabilizing element source, zirconia and a color reducing agent source. The compositions of the green body and the calcined body are not limited as long as the sintered body of the present embodiment is obtained, and may be similar to the composition of the target sintered body.

The raw material composition is, for example, a powder composition containing a stabilizing element source, zirconia and a color reducing agent source. Since the green body is composed of the raw material composition, the green body and the raw material composition have the same composition.

The stabilizing element source contains a cerium-containing compound (hereinafter may also be referred to as the "cerium source") and preferably contains a cerium source and a compound containing a sub-stabilizing element (hereinafter this compound may also be referred to as the "sub-stabilizing element source", and when the sub-stabilizing element is yttrium or the like, such a compound may be referred to as the "yttrium source" or the like). The cerium source and the sub-stabilizing element source are preferably respectively at least one selected from the group consisting of a chloride, a sulfide, a nitride, a hydroxide and an oxide of cerium and at least one selected from the group consisting of a chloride, a sulfide, a nitride, a hydroxide and an oxide of the sub-stabilizing element.

A more preferable stabilizing element source includes a cerium source and at least one selected from the group consisting of a magnesium source, a calcium source, an yttrium source, an erbium source and a scandium source, for example, a cerium source and at least one of an yttrium source and a magnesium source or a cerium source and an yttrium source.

The type of the stabilizing element and the content of the stabilizing element source in the raw material composition may be similar to those of the target green body and sintered body.

The color reducing agent source (hereinafter, when the color reducing agent is lanthanum or the like, the color reducing agent source may also be referred to as the "lanthanum source" or the like) is at least one of a compound of the element of the color reducing agent of the target sintered body and a compound of a precursor thereof, and an example thereof is a compound containing at least one selected from the group consisting of lanthanum, neodymium, samarium, europium, dysprosium, holmium, thulium, ytterbium and lutetium. The color reducing agent source is, for example, at least one selected from the group consisting of a chloride, a sulfate, a nitrate, a hydroxide, an oxalate, an acetate and an oxide and is preferably an oxide. For example, when lanthanum oxide ($La_2O_3$) is contained as the color reducing agent source, the green body may contain, as the lanthanum source, either or both of lanthanum oxide and a lanthanum-containing compound that serves as a precursor thereof. A specific example of the lanthanum source is at least one selected from the group consisting of lanthanum chloride, lanthanum sulfate, lanthanum nitrate, lanthanum hydroxide, lanthanum oxalate, lanthanum acetate and lanthanum oxide, and the lanthanum source is preferably lanthanum oxide. The content of the color reducing agent source in the raw material composition may be similar to the color reducing agent content of the target green body and sintered body.

The raw material composition may contain an additive component source. The additive component source is, for example, at least one selected from the group consisting of alumina, silica, gallium oxide, germanium oxide and a pigment.

The alumina source is either or both of alumina and an aluminum-containing compound that serves as a precursor thereof, and is, for example, at least one selected from the group consisting of aluminum chloride, aluminum sulfate, aluminum nitrate, aluminum hydroxide and alumina and is preferably alumina.

The raw material composition may contain a silica source. The silica source is either or both of silica and a silicon (Si)-containing compound that serves as a precursor thereof, and is, for example, at least one selected from the group consisting of quartz, silica sand, silica stone, silica sol and silica and is preferably silica.

The raw material composition may contain a gallium oxide source. The gallium oxide source is either or both of gallium oxide and a gallium-containing compound that serves as a precursor thereof, and is, for example, at least one selected from the group consisting of gallium oxide, gallium nitrate, gallium acetate and gallium hydroxide and is preferably gallium oxide.

The raw material composition may contain a germanium oxide source. The germanium oxide source is either or both of germanium oxide and an germanium-containing compound that serves as a precursor thereof, and is, for example, at least one selected from the group consisting of germanium oxide and germanium hydroxide and is preferably germanium oxide.

The content of the additive component such as the alumina source in the raw material composition may be similar to the additive component content such as the alumina content of the target sintered body.

The raw material composition may contain a pigment component source if necessary. The pigment component source may be either or both of a pigment and a precursor thereof. The precursor of the pigment is, for example, a compound that contains an element having a function of coloring zirconia, and is, for example, preferably a compound containing a metal element, such as at least one selected from the group consisting of an oxide, a hydroxide, an oxyhydroxide, a carbonate, an oxalate, a sulfate, an acetate, a nitrate, a chloride, a fluoride, a bromide and an iodide of a metal, and preferably at least one selected from the group consisting of an oxide, a hydroxide, an oxyhydroxide and a carbonate of a metal.

When the pigment is a metal complex oxide containing transition metals A and B and having a perovskite structure or a spinel structure, the pigment can be obtained by, for example, mixing at least one selected from the group consisting of an oxide, a hydroxide, an oxyhydroxide, a carbonate, an oxalate, a sulfate, an acetate, a nitrate, a chloride, a fluoride, a bromide and an iodide of the metal A with that of the metal B as necessary and firing the resulting mixture in an air atmosphere at 1200°C to 1500°C.

A commercially available product may be used as the pigment, and preferable examples of the pigment include at least one selected from the group consisting of $TiO_2$, $MnO_2$, $Fe_2O_3$, $CoAl_2O_4$, $Tb_2O_3$ and ZnO, $CoAl_2O_4$, $Fe_2O_3$ and ZnO.

The content of the pigment in the raw material composition may be similar to the content of the pigment component of the target sintered body.

The raw material composition may contain a binder for improving the shape stability. The binder may be any organic binder used in forming ceramics, and is, for example, at least one selected from the group consisting of an acrylic resin, a polyolefin resin, a wax and a plasticizer. A specific example of the binder is at least one selected from the group consisting of AS-1100, AS-1800 and AS-2000 (all trade names, produced by Toa Gosei Co., Ltd.). The content of the binder is, for example, 25 vol% or more and 65 vol% or less in terms of the proportion at which the binder occupies the volume of the raw material composition. For example, more than 0 mass% and 10 mass% or less of binder is contained in 100 mass% of the green body.

The shape of the green body may be any desired shape that suits the purpose and takes into account the sintering shrinkage, and is, for example, at least one selected from the group consisting of a spherical shape, an approximately spherical shape, an elliptical shape, a disk shape, a cylindrical shape, a cubic shape, a rectangular parallelepiped shape, a polyhedral shape and an approximately polyhedral shape.

The green body may be composed of a raw material composition containing a stabilizing element source, zirconia, a color reducing agent source and one or more additive component sources as necessary, and the raw material composition may be a compact that has a particular shape. The green body is obtained by forming a raw material composition containing a stabilizing element source, zirconia and a color reducing agent source and may be produced by any method. An example is to form a raw material composition composed of a mixed powder prepared by mixing a stabilizing element source, zirconia, a color reducing agent source and one or more additive component sources as necessary by any desired method. In addition, instead of or in addition to the stabilizing element source and zirconia, stabilizing element-containing zirconia may be used. The raw material composition used in producing the green body may have a composition similar to that of the target green body, and an example of the raw material composition is a powder of zirconia containing 0.05 mol% or more and 15 mol% or less of cerium as the stabilizing element and more than 0 mol% and 2 mol% or less of yttrium as oxide as the sub-stabilizing element. This powder can be used in a method for producing a sintered body characterized in using this powder, preferably, a method for producing the sintered body of the present embodiment characterized in using this powder.

When stabilizing element-containing zirconia is used as zirconia, the method of doping zirconia with the stabilizing element may be any. For example, a hydrated zirconia sol and a stabilizing element source in an amount similar to the target stabilizing element content are mixed, dried, calcined and washed with water. In mixing the components of the raw material composition, grinding may be employed. The grinding method may be any, may be at least one of wet grinding and dry grinding and is preferably wet grinding. A specific example of the wet grinding is using at least one selected from the group consisting of a ball mill, a vibrating mill and a continuous-type media stirring mill and preferably a ball mill.

The grinding conditions using a ball mill may involve, for example, mixing a calcined powder and a solvent to prepare a slurry in which the mass ratio of the calcined powder relative to the slurry mass is 30 mass% or more and 60 mass% or less and grinding the slurry for 10 hours or more and 100 hours or less or 10 hours or more and 30 hours or less by using zirconia balls having a diameter of 1 mm or more and 15 mm or less as the grinding media.

After wet grinding, drying may be performed by any method to prepare a powder. The drying conditions are, for example, an air atmosphere at 110°C to 130°C.

In order to improve the handling properties of the powder, the method for producing the powder of the present embodiment may include a step of granulating the powder (hereinafter this step may also be referred to as the "granulating step"). Granulation may be performed by any method and may involve, for example, forming granules by spraying a slurry in which the powder and a solvent are mixed. The solvent may be either or both of water and alcohol and is preferably water. The granulated powder (hereinafter may also be referred to as the "powder granules") has, for example, an average granule size of 30 $\mu$m or more and 80 $\mu$m or less or 50 $\mu$m or more and 60 $\mu$m or less and a bulk density of 1.00 g/cm$^3$ or more and 1.50 g/cm$^3$ or less or 1.10 g/cm$^3$ or more and 1.45 g/cm$^3$ or less.

A preferable example of the raw material composition is a powder of zirconia that contains a color reducing agent source and a cerium compound as the stabilizing element source, in which the cerium content is 0.05 mol% or more and 15 mol% or less and the color reducing agent content is 0.01 mass% or more and less than 1.95 mass%. Another example is a powder of zirconia containing a color reducing agent source, a cerium compound as a stabilizing element source and a compound of either or both of yttrium and magnesium, in which the cerium content is 0.05 mol% or more and 15 mol% or less and the color reducing agent content is 0.01 mass% or more and less than 1.95 mass%.

The forming method may be any known forming method that can form a compact from the raw material composition (mixed powder), is preferably at least one selected from the group consisting of uniaxial press-forming, isostatic press-forming, injection molding, extrusion molding, slip casting, tumbling granulation and slip casting, is more preferably either or both of uniaxial press-forming and an isostatic press-forming and yet more preferably either or both of cold isostatic pressing and uniaxial press-forming (powder press-forming).

A preferable example of the green body is a green body of zirconia containing a color reducing agent source and a cerium compound as a stabilizing element source, in which the cerium content is 0.05 mol% or more and 15 mol% or less and the color reducing agent content is 0.01 mass% or more and less than 1.95 mass%. Another example is a green body of zirconia containing a color reducing agent source and, as a stabilizing element source, a cerium compound and a compound of either or both of yttrium and magnesium, in which the cerium content is 0.05 mol% or more and 15 mol% or less and the color reducing agent content is 0.01 mass% or more and less than 1.95 mass%.

Prior to sintering the green body, a calcining step may be performed to prepare a calcined body. The calcining step may involve heat-treating the green body at a temperature lower than the sintering temperature of the powder and may involve, for example, heat-treating the green body in an air atmosphere at 800°C or more and less than 1100°C. In this manner, the powder particles form necks, and, as a result, a calcined body composed of particles in a fused state is obtained A preferable example of the calcined body is a calcined body of zirconia containing a color reducing agent source and a cerium compound as a stabilizing element source, in which the cerium content is 0.05 mol% or more and 15 mol% or less and the color reducing agent content is 0.01 mass% or more and less than 1.95 mass%. Another example is a calcined body of zirconia containing a color reducing agent source and, as a stabilizing element source, a cerium compound and a compound of either or both of yttrium and magnesium, in which the cerium content is 0.05 mol% or more and 15 mol% or less and the color reducing agent content is 0.01 mass% or more and less than 1.95 mass%.

The shape of the calcined body may be any desired shape that suits the purpose and takes into account the sintering shrinkage, and is, for example, at least one selected from the group consisting of a spherical shape, an approximately spherical shape, an elliptical shape, a disk shape, a cylindrical shape, a cubic shape, a rectangular parallelepiped shape, a polyhedral shape and an approximately polyhedral shape, in particular various shapes that correspond to applications.

In the sintering step, the green body or the calcined body is sintered into a sintered body. The sintering method may be any, and examples thereof include known sintering methods such as pressureless sintering, pressure sintering and vacuum sintering. A preferable sintering method is, for example, pressureless sintering, and for the purpose of convenience, the sintering method is preferably pressureless sintering only. In this manner, the sintered body of the present embodiment can be obtained as a so-called pressureless sintered body. Pressureless sintering refers to a method for sintering a material to be sintered (either or both of a green body and a calcined body) by simply heating the material without applying external force to the material during the sintering.

The sintering method is not particularly limited and is, for example, pressureless sintering in which the sintering atmosphere is an oxidizing atmosphere, in particular, an air atmosphere, and the sintering temperature is 1200°C or higher, 1300°C or higher or 1350°C or higher and lower than 1800°C, 1700°C or lower, 1600°C or lower or 1550°C or lower. A preferable sintering method is pressureless sintering in an air atmosphere at 1300°C or higher and 1650°C or lower, in particular, pressureless sintering in an air atmosphere at 1400°C or higher and 1600°C or lower or pressureless sintering in an air atmosphere at 1400°C or higher and 1575°C or lower. Note that, in this sintering method, there is a tendency in that the color tone becomes more achromatic and the light transmitting property is enhanced with the increase in the sintering temperature.

The powder or a green body thereof according to the present embodiment preferably has a sinterable temperature of 1200°C or higher, 1300°C or higher or 1350°C or higher and lower than 1800°C, 1700°C or lower, 1600°C or lower or 1550°C or lower.

The sinterable temperature is a sintering temperature at which the powder can be sintered, and that the powder can be sintered means that, when a powder is sintered according to a sintering profile in which the temperature is retained at a sintering temperature (hereinafter may also be referred to as the "retention temperature") that is the highest temperature during the sintering step, a sintered body that has a measured density of 5.50 g/cm$^3$ or more and is free of breaking or cracking is obtained. In other words, a powder is sinterable at 1500°C when a sintered body having a measured density of 5.50 g/cm$^3$ or more free of breaking or cracking is obtained from the powder by pressureless sintering in an air atmosphere at a heating rate of 100°C/hr, a retention temperature of 1500°C, a retention time of 2 hours, and a cooling rate of 200°C/hr.

Here, the details of the sintering atmosphere, the sintering method and the sintering profile are not particularly limited as long as the sintered body of the present embodiment is obtained, and the examples of the conditions are as follows.

Sintering atmosphere: oxidizing atmosphere, preferably, air atmosphere
Sintering method: pressureless sintering
Heating rate: 20°C/hr or more and 700°C/hr or less
Retention temperature: 1200°C or higher and lower than 1800°C
Retention time: more than 0 minutes but not more than 20 hours, preferably, 1 minute or more and 20 hours or less.
Cooling rate: 20°C/hr to 1000°C/hr

The green body, the calcined body and the sintered body of the present embodiment may be subjected to processing that suits the purpose at any time. Examples of the processing method include drilling, router processing, grinding, cutting and polishing. The influence of the shrinkage on the final product shape is smaller for the calcined body than for the green body and yet smaller for the sintered body than for the calcined body, and thus there is no need to consider the processing allowance design, and the processing loss can be reduced. Since the mechanical properties such as strength and hardness are improved, processing at higher speed becomes possible.

Another embodiment of the present disclosure is, for example, a powder of zirconia, the powder comprising a color reducing agent and cerium as a stabilizing element, in which the powder has a cerium content of 0.05 mol% or more and 15 mol% or less and a color reducing agent content of 0.01 mass% or more and less than 1.95 mass%.

Yet another embodiment of the present disclosure is a green body of zirconia, the green body comprising a color reducing agent and cerium as a stabilizing element, in which the green body has a cerium content of 0.05 mol% or more and 15 mol% or less and a color reducing agent content of 0.01 mass% or more and less than 1.95 mass%.

Yet another embodiment of the present disclosure is a calcined body of zirconia, the calcined body comprising a color reducing agent and cerium as a stabilizing element, in which the calcined body has a cerium content of 0.05 mol% or more and 15 mol% or less and a color reducing agent content of 0.01 mass% or more and less than 1.95 mass%.

EXAMPLES

Hereinafter, the present disclosure is specifically described through examples. However, these examples do not limit the present disclosure.

(BET specific surface area)

The BET specific surface area of a powder sample was measured with a common flow-type specific surface area automatic analyzer (instrument name: FlowSorb III2305 produced by Shimadzu Corporation) using nitrogen as an adsorption gas. Prior to the measurement, the powder sample was subjected to a pretreatment involving deaeration treatment in an air atmosphere at 250°C for 30 minutes.

(Sintered body density)

The measured density of the sintered body sample was determined as a ratio (g/cm$^3$) of the mass measured by mass measurement relative to the volume determined by the Archimedes' method compliant with JIS R 1634.

(Vickers hardness)

The Vickers hardness was measured with a common Vickers hardness tester (instrument name: Q-30A produced by Verder Scientific GmbH) equipped with a square pyramid diamond indenter.

The indenter was statically pressed into a measurement sample surface, and the diagonal length of the indentation mark formed in the measurement sample surface was measured with naked eye. The obtained diagonal length was used to determine the Vickers hardness (GPa) from the equation described above.

A disk-shaped sintered body 20 mm diameter ×1 mm thickness was mirror-polished and used as the measurement sample.

(Fracture toughness)

The fracture toughness value of the sintered body sample was measured by a method that conforms with the SEPB method compliant with JIS R 1607.

(Flexural strength)

The flexural strength of the sintered body sample was measured by a three-point flexural test compliant with JIS R 1601.

(Measuring color tone)

The color tone of the sintered body sample was measured in accordance with JIS Z 8722. A common spectrophotometric colorimeter (instrument name: CM-700d produced by KONICA MINOLTA JAPAN, INC.) was used in measurement in a black background measurement mode using a black back plate. The measurement conditions were as follows.

Light source: D-65 light source
Viewing angle: 10°
Measurement system: SCI

A disk-shaped sintered body 20 mm diameter ×1 mm thickness was mirror-polished and used as the sintered body sample. The color tone evaluation effective area was a diameter of 10 mm.

(Total luminous transmittance)

The total luminous transmittance was measured with a haze meter (instrument name: NDH4000 produced by NIPPON DENSHOKU INDUSTRIES Co., Ltd.) using a D65 light source by a method compliant with JIS K 7361-1. The measurement sample was a disk-shaped sintered body having both surfaces polished to surface roughness Ra ≤ 0.02 μm and having a thickness of 1.0 ± 0.1 mm.

Example 1

An aqueous zirconium oxychloride solution was hydrolyzed to obtain a hydrated zirconia sol. Cerium chloride heptahydrate and yttrium chloride were added to and mixed with the hydrated zirconia sol such that the cerium amount was 4.3 mol% and the yttrium content was 0.98 mol%. After mixing, the resulting mixture was dried in an air atmosphere and calcined in an air atmosphere at 1000°C for 2 hours to obtain a cerium-and-yttrium-stabilized zirconia calcined powder. The obtained calcined powder was washed with pure water and dried, and La$_2$O$_3$ was added thereto as a color reducing agent such that the La$_2$O$_3$ content was 0.1 mass% to obtain a mixed powder. The mixed powder was added to pure water to prepare slurry, and the slurry was ground and mixed in a ball mill with zirconia balls 2 mm in diameter as the grinding media for 16 hours. The slurry after grinding and mixing was dried, as a result of which a powder of this example composed of cerium-and-yttrium-stabilized zirconia having a cerium amount of 4.3 mol%, an yttrium content of 0.98 mol% and a La$_2$O$_3$ content of 0.1 mass% was obtained.

The obtained powder was packed in a cylindrical die having a diameter of 25 mm, uniaxially pressed at a forming pressure of 50 MPa and subjected to cold isostatic pressing (CIP) treatment at a forming pressure of 196 MPa to obtain a cylindrical green body. The obtained green body was sintered under the following conditions, as a result of which a sintered body of this example composed of cerium-and-yttrium-stabilized zirconia having a cerium amount of 4.3 mol%, an yttrium content of 0.98 mol% and a $La_2O_3$ content of 0.1 mass% was obtained.

    Sintering method: pressureless sintering
    Sintering atmosphere: air atmosphere
    Sintering temperature: 1550°C
    Sintering time: 2 hours

Example 2

A sintered body of this example was obtained as in Example 1 except that the $La_2O_3$ content was changed to 0.25 mass%.

Example 3

A sintered body of this example was obtained as in Example 1 except that the $La_2O_3$ content was changed to 0.5 mass%.

Example 4

A sintered body of this example was obtained as in Example 3 except that the obtained powder was packed in a 40 mm length × 30 mm width plate-shaped die, uniaxially pressed at a forming pressure of 50 MPa and subjected to cold isostatic pressing (CIP) treatment at a forming pressure of 196 MPa to obtain a plate-shaped green body and that the sintering temperature was changed 1500°C. The plate-shaped sintered body was processed and measured to determine the three-point flexural strength and the fracture toughness value defined by the SEPB method, and the average three-point flexural strength was 858 MPa and the fracture toughness value was 9.7.

Examples 5 to 8

Sintered bodies of these examples were obtained as in Example 3 except that the grinding time was changed to 24 hours and that the sintering temperature was changed to 1400°C (Example 5), 1450°C (Example 6), 1500°C (Example 7) and 1550°C (Example 8).

Examples 9 and 10

Sintered bodies of these examples were obtained as in Example 1 except that the $La_2O_3$ content was changed to 0.75 mass% and that the sintering temperature was changed to 1450°C (Example 9) and 1500°C (Example 10).

Example 11

An aqueous zirconium oxychloride solution was hydrolyzed to obtain a hydrated zirconia sol. Cerium chloride heptahydrate and yttrium chloride were added to and mixed with the hydrated zirconia sol such that the cerium amount was 4.3 mol% and the yttrium content was 0.98 mol%. After mixing, the resulting mixture was dried in an air atmosphere and calcined in an air atmosphere at 1000°C for 2 hours to obtain a cerium-and-yttrium-stabilized zirconia calcined powder. The obtained calcined powder was washed with pure water and dried, $La_2O_3$ was added thereto as a color reducing agent so that the $La_2O_3$ content was 0.5 mass%, $Al_2O_3$ was further added thereto as an additive component so that the $Al_2O_3$ content was 0.05 mass%, the resulting mixed powder was added to pure water to prepare a slurry, and the slurry was ground and mixed in a ball mill with zirconia balls having a diameter of 2 mm as grinding media for 16 hours. The slurry after grinding and mixing was dried, as a result of which a powder of this example composed of cerium-and-yttrium-stabilized zirconia having a cerium amount of 4.3 mol%, an yttrium content of 0.98 mol%, a $La_2O_3$ content of 0.5 mass% and a $La_2O_3$ content of 0.05 mass% was obtained.

The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this example. The obtained green body was sintered under the following conditions, as a result of which a sintered body of this example composed of cerium-and-yttrium-stabilized zirconia having a cerium amount of 4.3 mol%, an yttrium content of 0.98 mol%, a $La_2O_3$ content of 0.5 mass% and an $Al_2O_3$ content of 0.05 mass% was obtained.

Sintering method: pressureless sintering
Sintering atmosphere: air atmosphere
Sintering temperature: 1400°C
Sintering time: 2 hours

Example 12

A sintered body of this example was obtained as in Example 11 except that the sintering temperature was changed to 1450°C.

Example 13

A sintered body of this example was obtained as in Example 11 except that the obtained powder was packed in a 40 mm length × 30 mm width plate-shaped die, uniaxially pressed at a forming pressure of 50 MPa and subjected to cold isostatic pressing (CIP) treatment at a forming pressure of 196 MPa to obtain a plate-shaped green body and that the sintering temperature was changed 1500°C.
The plate-shaped sintered body was processed and measured to determine the three-point flexural strength and the fracture toughness value defined by the SEPB method, and the average three-point flexural strength was 912 MPa and the fracture toughness value was 10.8.

Example 14

A sintered body of this example was obtained as in Example 11 except that the obtained powder was packed in a 40 mm length × 30 mm width plate-shaped die, uniaxially pressed at a forming pressure of 50 MPa and subjected to cold isostatic pressing (CIP) treatment at a forming pressure of 196 MPa to obtain a plate-shaped green body and that the sintering temperature was changed 1550°C. The plate-shaped sintered body was processed and measured to determine the three-point flexural strength and the fracture toughness value defined by the SEPB method, and the average three-point flexural strength was 814 MPa and the fracture toughness value was 12.0.

Examples 15 to 17

A sintered body of each example was obtained as in Example 11 except that the $Al_2O_3$ content was changed to 0.1 mass% and that the sintering temperature was changed to 1350°C (Example 15), 1400°C (Example 16) and 1450°C (Example 17).

Example 18

An aqueous zirconium oxychloride solution was hydrolyzed to obtain a hydrated zirconia sol. Cerium chloride heptahydrate and yttrium chloride were added to and mixed with the hydrated zirconia sol such that the cerium amount was 7.2 mol% and the yttrium content was 1.0 mol%. After mixing, the resulting mixture was dried in an air atmosphere and calcined in an air atmosphere at 1000°C for 2 hours to obtain a cerium-and-yttrium-stabilized zirconia calcined powder. The obtained calcined powder was washed with pure water and dried, $La_2O_3$ was added thereto as a color reducing agent so that the $La_2O_3$ content was 0.5 mass%, the resulting mixed powder was added to pure water to prepare a slurry, and the slurry was ground and mixed in a ball mill with zirconia balls having a diameter of 2 mm as grinding media for 24 hours. The slurry after grinding and mixing was dried, as a result of which a powder of this example composed of cerium-and-yttrium-stabilized zirconia having a cerium amount of 7.2 mol%, an yttrium content of 1.0 mol% and a $La_2O_3$ content of 0.5 mass% was obtained.
The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this example. The obtained green body was sintered under the following conditions, a cerium amount of 7.2 mol%, an yttrium content of 1.0 mol% and a $La_2O_3$ content of 0.5 mass%

Sintering method: pressureless sintering
Sintering atmosphere: air atmosphere
Sintering temperature: 1550°C
Sintering time: 2 hours

Example 19

A sintered body of this example was obtained as in Example 18 except that the $La_2O_3$ content was changed to 1.0 mass%.

Comparative example 1

An aqueous zirconium oxychloride solution was hydrolyzed to obtain a hydrated zirconia sol. Cerium chloride heptahydrate and yttrium chloride were added to and mixed with the hydrated zirconia sol such that the cerium amount was 4.3 mol% and the yttrium content was 0.98 mol%. After mixing, the resulting mixture was dried in an air atmosphere and calcined in an air atmosphere at 1000°C for 2 hours to obtain a calcined powder of cerium-and-yttrium-stabilized zirconia. The obtained calcined powder was washed with pure water and dried and then added to pure water to prepare slurry, and the slurry was ground and mixed in a ball mill with zirconia balls 2 mm in diameter as the grinding media for 16 hours. The slurry after grinding and mixing was dried, as a result of which a powder of this comparative example composed of cerium-and-yttrium-stabilized zirconia having a cerium amount of 4.3 mol%, an yttrium content of 0.98 mol% was obtained.

The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this comparative example. The obtained green body was sintered under the following conditions, as a result of which a sintered body of this comparative example composed of cerium-and-yttrium-stabilized zirconia having a cerium amount of 4.3 mol% and an yttrium content of 0.98 mol% was obtained.

    Sintering method: pressureless sintering
    Sintering atmosphere: air atmosphere
    Sintering temperature: 1550°C
    Sintering time: 2 hours

Comparative example 2

A sintered body of this comparative example was obtained as in Comparative example 1 except that the grinding time was changed to 24 hours.

Comparative example 3

An aqueous zirconium oxychloride solution was hydrolyzed to obtain a hydrated zirconia sol. Cerium chloride heptahydrate and yttrium chloride were added to and mixed with the hydrated zirconia sol such that the cerium amount was 4.3 mol% and the yttrium content was 0.98 mol%. After mixing, the resulting mixture was dried in an air atmosphere and calcined in an air atmosphere at 1000°C for 2 hours to obtain a cerium-and-yttrium-stabilized zirconia calcined powder. The obtained calcined powder was washed with pure water and dried, $Al_2O_3$ was then added thereto as an additive component so that the $Al_2O_3$ content was 0.05 mass%, the resulting mixed powder was added to pure water to prepare a slurry, and the slurry was ground and mixed in a ball mill with zirconia balls having a diameter of 2 mm as grinding media for 16 hours. The slurry after grinding and mixing was dried, as a result of which a powder of this comparative example composed of cerium-and-yttrium-stabilized zirconia having a cerium amount of 4.3 mol%, an yttrium content of 0.98 mol% and an $Al_2O_3$ content of 0.05 mass% was obtained.

The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this comparative example. The obtained green body was sintered under the following conditions, as a result of which a sintered body of this comparative example composed of cerium-and-yttrium-stabilized zirconia having a cerium amount of 4.3 mol%, an yttrium content of 0.98 mol% and a $Al_2O_3$ content of 0.05 mass% was obtained.

    Sintering method: pressureless sintering
    Sintering atmosphere: air atmosphere
    Sintering temperature: 1400°C
    Sintering time: 2 hours

Comparative example 4

A sintered body of this comparative example was obtained as in Comparative example 3 except that the sintering temperature was changed to 1450°C.

Comparative example 5

A sintered body of this comparative example was obtained as in Comparative example 3 except that the obtained powder was packed in a 40 mm length × 30 mm width plate-shaped die, uniaxially pressed at a forming pressure of 50 MPa and subjected to cold isostatic pressing (CIP) treatment at a forming pressure of 196 MPa to obtain a plate-shaped green body and that the sintering temperature was changed 1500°C.

Comparative example 6

A sintered body of this comparative example was obtained as in Comparative example 3 except that the obtained powder was packed in a 40 mm length × 30 mm width plate-shaped die, uniaxially pressed at a forming pressure of 50 MPa and subjected to cold isostatic pressing (CIP) treatment at a forming pressure of 196 MPa to obtain a plate-shaped green body and that the sintering temperature was changed 1550°C. The plate-shaped sintered body was processed and measured to determine the three-point flexural strength and the fracture toughness value defined by the SEPB method, and the average three-point flexural strength was 781 MPa and the fracture toughness value was 11.2.

Comparative example 7

A sintered body of this comparative example was obtained as in Example 1 except that the $La_2O_3$ content was changed to 2.0 mass%.

Comparative example 8

An aqueous zirconium oxychloride solution was hydrolyzed to obtain a hydrated zirconia sol. Yttrium chloride was added to and mixed with the hydrated zirconia sol such that the yttrium content was 3 mol%. After mixing, the resulting mixture was dried in an air atmosphere and calcined in an air atmosphere at 1000°C for 2 hours to obtain an yttrium-stabilized zirconia calcined powder. The obtained calcined powder was washed with pure water and dried, $Al_2O_3$ was then added thereto as an additive component so that the $Al_2O_3$ content was 0.05 mass%, the resulting mixed powder was added to pure water to prepare a slurry, and the slurry was ground and mixed in a ball mill with zirconia balls having a diameter of 2 mm as grinding media for 16 hours. The slurry after grinding and mixing was dried, as a result of which a powder of this comparative example composed of yttrium-stabilized zirconia having an yttrium content of 3 mol% and an $Al_2O_3$ content of 0.05 mass% was obtained.
The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this comparative example. The obtained green body was sintered under the following conditions, as a result of which a sintered body of this comparative example composed of yttrium-stabilized zirconia having an yttrium content of 3 mol% and an $Al_2O_3$ content of 0.05 mass% was obtained.

Sintering method: pressureless sintering
Sintering atmosphere: air atmosphere
Sintering temperature: 1450°C
Sintering time: 2 hours

Comparative example 9

A zirconia sintered body of this comparative example was obtained as in Comparative example 8 except that the yttrium content was 5.5 mol%.

Comparative example 10

An aqueous zirconium oxychloride solution was hydrolyzed to obtain a hydrated zirconia sol. Cerium chloride was added to and mixed with the hydrated zirconia sol such that the cerium amount was 4.95 mol%. After mixing, the resulting mixture was dried in an air atmosphere and calcined in an air atmosphere at 1000°C for 2 hours to obtain an cerium-stabilized zirconia calcined powder. The obtained calcined powder was washed with pure water and dried, $La_2O_3$ was added thereto as a color reducing agent so that the $La_2O_3$ content was 0.5 mass%, the resulting mixed powder was added to pure water to prepare a slurry, and the slurry was ground and mixed in a ball mill with zirconia balls having a diameter of 2 mm as grinding media for 16 hours. The slurry after grinding and mixing was dried, as a result of which a powder of this comparative example composed of cerium-stabilized zirconia having a cerium amount of 4.95 mol% and a

La$_2$O$_3$ content of 0.5 mass% was obtained.

The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this comparative example. The obtained green body was sintered under the following conditions, as a result of which a sintered body of this comparative example composed of cerium-stabilized zirconia having a cerium amount of 4.95 mol% and a La$_2$O$_3$ content of 0.5 mass% was obtained.

Sintering method: pressureless sintering
Sintering atmosphere: air atmosphere
Sintering temperature: 1350°C
Sintering time: 2 hours

Comparative example 11

A sintered body of this comparative example was obtained as in Comparative example 10 except that the sintering temperature was changed to 1400°C.

Comparative example 12

A sintered body of this comparative example was obtained as in Comparative example 10 except that the sintering temperature was changed to 1450°C.

The powders of Examples and Comparative examples described above are as follows.

[Table 1]

| | CeO$_2$ (mol%) | Y$_2$O$_3$ (mol%) | Y$_2$O$_3$ /CeO$_2$ (mol/mol) | Stabilizing element amount (mol%) | La$_2$O$_3$ (mass%) | Al$_2$O$_3$ (mass%) | BET (m$^2$/g) |
|---|---|---|---|---|---|---|---|
| Example 1 | 4.3 | 0.98 | 0.23 | 5.28 | 0.1 | 0 | 11.8 |
| Example 2 | 4.3 | 0.98 | 0.23 | 5.28 | 0.25 | 0 | 12.8 |
| Example 3 | 4.3 | 0.98 | 0.23 | 5.28 | 0.5 | 0 | 11.8 |
| Example 4 | 4.3 | 0.98 | 0.23 | 5.28 | 0.5 | 0 | 11.8 |
| Example 5 | 4.3 | 0.98 | 0.23 | 5.28 | 0.5 | 0 | 13.2 |
| Example 6 | 4.3 | 0.98 | 0.23 | 5.28 | 0.5 | 0 | 13.2 |
| Example 7 | 4.3 | 0.98 | 0.23 | 5.28 | 0.5 | 0 | 13.2 |
| Example 8 | 4.3 | 0.98 | 0.23 | 5.28 | 0.5 | 0 | 13.2 |
| Example 9 | 4.3 | 0.98 | 0.23 | 5.28 | 0.75 | 0 | 13.1 |
| Example 10 | 4.3 | 0.98 | 0.23 | 5.28 | 0.75 | 0 | 13.1 |
| Example 11 | 4.3 | 0.98 | 0.23 | 5.28 | 0.5 | 0.05 | 12.6 |
| Example 12 | 4.3 | 0.98 | 0.23 | 5.28 | 0.5 | 0.05 | 12.6 |
| Example 13 | 4.3 | 0.98 | 0.23 | 5.28 | 0.5 | 0.05 | 12.6 |
| Example 14 | 4.3 | 0.98 | 0.23 | 5.28 | 0.5 | 0.05 | 12.6 |
| Example 15 | 4.3 | 0.98 | 0.23 | 5.28 | 0.5 | 0.1 | 14.7 |
| Example 16 | 4.3 | 0.98 | 0.23 | 5.28 | 0.5 | 0.1 | 14.7 |
| Example 17 | 4.3 | 0.98 | 0.23 | 5.28 | 0.5 | 0.1 | 14.7 |
| Example 18 | 7.2 | 1.0 | 0.14 | 8.20 | 0.5 | 0 | 16.9 |
| Example 19 | 7.2 | 1.0 | 0.14 | 8.20 | 1 | 0 | 16.6 |
| Comparative example 1 | 4.3 | 0.98 | 0.23 | 5.28 | 0 | 0 | 11.5 |
| Comparative example 2 | 4.3 | 0.98 | 0.23 | 5.28 | 0 | 0 | 13.1 |

(continued)

| | CeO$_2$ (mol%) | Y$_2$O$_3$ (mol%) | Y$_2$O$_3$ /CeO$_2$ (mol/mol) | Stabilizing element amount (mol%) | La$_2$O$_3$ (mass%) | Al$_2$O$_3$ (mass%) | BET (m$^2$/g) |
|---|---|---|---|---|---|---|---|
| Comparative example 3 | 4.3 | 0.98 | 0.23 | 5.28 | 0 | 0.05 | 12.3 |
| Comparative example 4 | 4.3 | 0.98 | 0.23 | 5.28 | 0 | 0.05 | 12.3 |
| Comparative example 5 | 4.3 | 0.98 | 0.23 | 5.28 | 0 | 0.05 | 12.3 |
| Comparative example 6 | 4.3 | 0.98 | 0.23 | 5.28 | 0 | 0.05 | 12.3 |
| Comparative example 7 | 4.3 | 0.98 | 0.23 | 5.28 | 2 | 0 | 11.9 |
| Comparative example 8 | 0.0 | 3.0 | - | 3.00 | 0 | 0.05 | 13.0 |
| Comparative example 9 | 0.0 | 5.5 | - | 5.50 | 0 | 0.05 | 10.0 |
| Comparative example 10 | 4.95 | 0 | 0.00 | 4.95 | 0.5 | 0 | 14.6 |
| Comparative example 11 | 4.95 | 0 | 0.00 | 4.95 | 0.5 | 0 | 14.6 |
| Comparative example 12 | 4.95 | 0 | 0.00 | 4.95 | 0.5 | 0 | 14.6 |

The sintered bodies of Examples and Comparative examples described above are as follows.

[Table 2]

| | Sintering temperature (°C) | Sintered body density (g/cm$^3$) | L* | a* | b* | C* | Hardness Hv10 | Total luminous transmittance (%) |
|---|---|---|---|---|---|---|---|---|
| Example 1 | 1550 | 6.17 | 81.43 | -3.05 | 7.64 | 8.2 | 1006 | 37.2 |
| Example 2 | 1550 | 6.19 | 77.91 | -2.16 | 3.11 | 3.8 | 1023 | 44.5 |
| Example 3 | 1550 | 6.18 | 78.82 | -1.76 | 4.46 | 4.8 | 1098 | 41.9 |
| Example 4 | 1500 | 6.19 | 78.45 | -1.41 | 2.64 | 3.0 | 1040 | 42.3 |
| Example 5 | 1400 | 6.17 | 81.22 | -3.34 | 6.65 | 7.4 | 1158 | 40.7 |
| Example 6 | 1450 | 6.18 | 80.31 | -2.78 | 5.75 | 6.4 | 1152 | 42.0 |
| Example 7 | 1500 | 6.19 | 78.68 | -2.11 | 4.82 | 5.3 | 1108 | 44.1 |
| Example 8 | 1550 | 6.20 | 77.43 | -1.62 | 3.04 | 3.4 | 1067 | 44.5 |
| Example 9 | 1450 | 6.16 | 80.62 | -2.76 | 8.20 | 8.7 | 1134 | 37.6 |
| Example 10 | 1500 | 6.18 | 78.72 | -2.42 | 6.47 | 6.9 | 1102 | 40.2 |
| Example 11 | 1400 | 6.17 | 81.17 | -2.44 | 4.06 | 4.7 | 1167 | 41.3 |
| Example 12 | 1450 | 6.18 | 80.36 | -2.11 | 3.51 | 4.1 | 1152 | 42.3 |
| Example 13 | 1500 | 6.19 | 79.32 | -1.67 | 2.81 | 3.3 | 1105 | 42.4 |
| Example 14 | 1550 | 6.20 | 79.56 | -1.50 | 1.97 | 2.5 | 1079 | 42.4 |
| Example 15 | 1350 | 6.16 | 85.25 | -2.10 | 4.09 | 4.6 | 1172 | 30.7 |
| Example 16 | 1400 | 6.16 | 83.92 | -1.97 | 3.47 | 4.0 | 1184 | 33.4 |
| Example 17 | 1450 | 6.16 | 82.70 | -1.64 | 2.94 | 3.4 | 1125 | 35.4 |
| Example 18 | 1550 | 6.24 | 77.84 | -2.08 | 2.93 | 3.6 | 1152 | 42.0 |
| Example 19 | 1550 | 6.24 | 77.54 | -4.57 | 10.13 | 11.1 | 1155 | 44.5 |
| Comparative example 1 | 1500 | 6.17 | 81.22 | -5.58 | 20.84 | 21.6 | 1000 | 36.3 |

(continued)

| | Sintering temperature (°C) | Sintered body density (g/cm$^3$) | L* | a* | b* | C* | Hardness Hv10 | Total luminous transmittance (%) |
|---|---|---|---|---|---|---|---|---|
| Comparative example 2 | 1500 | 6.17 | 77.60 | -5.91 | 24.32 | 25.0 | 1000 | 42.2 |
| Comparative example 3 | 1400 | 6.17 | 78.69 | -5.69 | 20.81 | 21.6 | 1149 | 39.7 |
| Comparative example 4 | 1450 | 6.18 | 78.53 | -5.48 | 17.90 | 18.7 | 1113 | 40.6 |
| Comparative example 5 | 1500 | 6.18 | 79.32 | -4.85 | 13.91 | 14.7 | 1029 | 39.9 |
| Comparative example 6 | 1550 | 6.19 | 81.07 | -4.46 | 11.46 | 12.3 | 1001 | 38.0 |
| Comparative example 7 | 1550 | 6.19 | 80.20 | -6.95 | 15.25 | 16.8 | 1058 | 40.3 |
| Comparative example 8 | 1450 | 6.09 | 82.53 | -0.64 | -2.44 | 2.5 | 1250 | 41.0 |
| Comparative example 9 | 1450 | 6.05 | 77.05 | -0.86 | -3.28 | 3.4 | 1250 | 49.0 |
| Comparative example 10 | 1350 | | - | - | - | - | - | - |
| Comparative example 11 | 1400 | | - | - | - | - | - | - |
| Comparative example 12 | 1450 | | - | - | - | - | - | - |

In the L*a*b* ranges of the sintered bodies of Examples, the color tone was white to milky white, L* was 77.0 or more and C* was 12.0 or less. In addition, the total luminous transmittance was 20% or more or even 40% or more, and this shows that the sintered bodies had high aesthetic merit as well as high transmittance suitable for use as the dental materials. Moreover, since the hardness HV10 of the sintered bodies was as low as lower than 1200, the processability was high, and the sintered bodies were suitable for sintered body processing.

Sintered bodies of Comparative examples 1 to 6, which did not contain a color reducing agent, had a strong yellow tone derived from cerium. In Comparative example 7, the color reducing agent content was excessively high, and thus the yellow tone could not be reduced appropriately and the sintered body had low aesthetic merit.

The sintered bodies of Comparative examples 8 and 9 do not contain cerium and are stabilized by yttrium only, and the hardness Hv10 was 1250. Thus, it could be confirmed that the sintered bodies had low processability.

Comparative examples 10 to 12 provided sintered bodies of cerium-stabilized zirconia containing only cerium as the stabilizing element. However, since the stabilizing element amount was low, densification did not proceed, cracking occurred, and sintered bodies could not be obtained.

Example 20

A powder and a sintered body of this example composed of cerium-and-yttrium-stabilized zirconia having a cerium amount of 3.0 mol%, an yttrium content of 1.1 mol%, a $La_2O_3$ content of 0.5 mass% and an $Al_2O_3$ content of 0.05 mass% was obtained as in Example 1 except that cerium chloride heptahydrate and yttrium chloride were added to and mixed with the hydrated zirconia sol such that the cerium amount was 3.0 mol% and the yttrium content was 1.1 mol%.

Example 21

A cerium-and-yttrium-stabilized zirconia calcined powder was obtained as in Example 1 except that cerium chloride heptahydrate and yttrium chloride were added to and mixed with the hydrated zirconia sol such that the cerium amount was 3.9 mol% and the yttrium content was 0.7 mol%. A powder of this example composed of cerium-and-yttrium-stabilized zirconia having a cerium amount of 3.9 mol%, an yttrium content of 0.7 mol%, a neodymium content of 0.5 mass% and an $Al_2O_3$ content of 0.05 mass% was obtained as in Example 1 except that, after the obtained calcined powder was washed with pure water and dried, neodymium oxide was added as a color reducing agent such that the neodymium content was 0.5 mass% and $Al_2O_3$ was further added thereto as an additive component such that the $Al_2O_3$ content was 0.05 mass%.

The powder of this example was sintered as in Example 1, as a result of which a sintered body of this example composed of cerium-and-yttrium-stabilized zirconia having a cerium amount of 3.9 mol%, an yttrium content of 0.7 mol%, a $Nd_2O_3$ content of 0.5 mass% and an $Al_2O_3$ content of 0.05 mass% was obtained.

[Table 3]

| | $CeO_2$ (mol%) | $Y_2O_3$ (mol%) | Sub-stabilizing element /$CeO_2$ (mol/mol) | Stabilizing element amount (mol%) | $La_2O_3$ (mass%) | $Al_2O_3$ (mass%) | BET ($m^2$/g) |
|---|---|---|---|---|---|---|---|
| Example 20 | 3.0 | 1.1 | 0.37 | 4.10 | 0.5 | 0.05 | 16.2 |
| Example 21 | 3.9 | 0.7 | 0.18 | 4.60 | 0.5 | 0.05 | 15.1 |

The sintered bodies of Examples described above are indicated in the table below.

[Table 4]

| | Sintering temperature (°C) | Sintered body density (g/cm³) | L* | a* | b* | C* | Hardness Hv10 | Total luminous transmittance (%) |
|---|---|---|---|---|---|---|---|---|
| Example 20 | 1550 | 6.14 | 78.55 | -1.18 | 2.16 | 2.5 | 999 | 40.6 |
| Example 21 | 1550 | 6.17 | 75.84 | -2.46 | 0.46 | 2.5 | 1055 | 30.0 |

It could be confirmed that the sintered body stabilized with cerium and yttrium with a reduced cerium amount in Example 20 was a sintered body having high aesthetic merit and a white to milky white color tone as well as high transmittance. It could also be confirmed that the sintered body containing neodymium as a color reducing agent in Example 21 was a sintered body having high aesthetic merit and a white to milky white color tone as well as high transmittance.

Example 22

An aqueous zirconium oxychloride solution was hydrolyzed to obtain a hydrated zirconia sol. Cerium chloride heptahydrate and magnesium chloride were added to and mixed with the hydrated zirconia sol such that the cerium amount was 7.0 mol% and the magnesium content was 1.0 mol%. After mixing, the resulting mixture was dried in an air atmosphere and calcined in an air atmosphere at 1000°C for 2 hours to obtain an cerium-and-magnesium stabilized zirconia calcined powder. The obtained calcined powder was washed with pure water and dried, $La_2O_3$ was added thereto as a color reducing agent so that the $La_2O_3$ content was 0.5 mass%, the resulting mixed powder was added to pure water to prepare a slurry, and the slurry was ground and mixed in a ball mill with zirconia balls having a diameter of 2 mm as grinding media for 16 hours. The slurry after grinding and mixing was dried, as a result of which a powder of this example composed of cerium-and-magnesium-stabilized zirconia having a cerium amount of 7.0 mol%, a magnesium content of 1.0 mol% and a $La_2O_3$ content of 0.5 mass% was obtained.

The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this example. The obtained green body was sintered under the following conditions, as a result of which a sintered body of this example composed of cerium-and-magnesium-stabilized zirconia having a cerium amount of 7.0 mol%, a magnesium content of 1.0 mol% and a $La_2O_3$ content of 0.5 mass% was obtained.

Sintering method: pressureless sintering

Sintering atmosphere: air atmosphere
Sintering temperature: 1400°C
Sintering time: 2 hours

Example 23

A sintered body of this example was obtained as in Example 22 except that the sintering temperature was changed to 1450°C.

Example 24

An aqueous zirconium oxychloride solution was hydrolyzed to obtain a hydrated zirconia sol. Cerium chloride heptahydrate and magnesium chloride were added to and mixed with the hydrated zirconia sol such that the cerium amount was 7.0 mol% and the magnesium content was 1.0 mol%. After mixing, the resulting mixture was dried in an air atmosphere and calcined in an air atmosphere at 1000°C for 2 hours to obtain an cerium-and-magnesium stabilized zirconia calcined powder. The obtained calcined powder was washed with pure water and dried, $La_2O_3$ was added thereto as a color reducing agent so that the $La_2O_3$ content was 0.5 mass%, $Al_2O_3$ was further added thereto as an additive component so that the $Al_2O_3$ content was 0.05 mass%, the resulting mixed powder was added to pure water to prepare a slurry, and the slurry was ground and mixed in a ball mill with zirconia balls having a diameter of 2 mm as grinding media for 16 hours. The slurry after grinding and mixing was dried, as a result of which a powder of this example composed of cerium-and-magnesium-stabilized zirconia having a cerium amount of 7.0 mol%, a magnesium content of 1.0 mol%, a $La_2O_3$ content of 0.5 mass% and a $Al_2O_3$ content of 0.05 mass% was obtained.

The obtained powder was packed in a cylindrical die having a diameter of 25 mm, uniaxially pressed at a forming pressure of 50 MPa and subjected to cold isostatic pressing treatment at a forming pressure of 196 MPa to obtain a cylindrical green body of this example. The obtained green body was sintered under the following conditions, as a result of which a zirconia sintered body of this example composed of cerium-and-magnesium-stabilized zirconia having a cerium amount of 7.0 mol%, a magnesium content of 1.0 mol%, a $La_2O_3$ content of 0.5 mass% and an $Al_2O_3$ content of 0.05 mass% was obtained.

Sintering method: pressureless sintering
Sintering atmosphere: air atmosphere
Sintering temperature: 1350°C
Sintering time: 2 hours

Examples 25 and 26

Sintered bodies of these examples were obtained as in Example 24 except that the sintering temperature was changed to 1400°C (Example 25) and 1450°C (Example 26).

The powders of Examples described above are indicated in the table below.

[Table 5]

| | $CeO_2$ (mol%) | MgO (mol%) | $MgO/CeO_2$ (mol/mol) | Stabilizing element amount (mol%) | $La_2O_3$ (mass%) | $Al_2O_3$ (mass%) | BET ($m^2/g$) |
|---|---|---|---|---|---|---|---|
| Example 22 | 7.0 | 1.0 | 0.14 | 8.00 | 0.5 | 0 | 18.0 |
| Example 23 | 7.0 | 1.0 | 0.14 | 8.00 | 0.5 | 0 | 18.0 |
| Example 24 | 7.0 | 1.0 | 0.14 | 8.00 | 0.5 | 0.05 | 17.0 |
| Example 25 | 7.0 | 1.0 | 0.14 | 8.00 | 0.5 | 0.05 | 17.0 |
| Example 26 | 7.0 | 1.0 | 0.14 | 8.00 | 0.5 | 0.05 | 17.0 |

The sintered bodies of Examples described above are indicated in the table below.

[Table 6]

| | Sintering temperature (°C) | Sintered body density (g/cm³) | L* | a* | b* | C* | Hardness Hv10 | Total luminous transmittance (%) |
|---|---|---|---|---|---|---|---|---|
| Example 22 | 1400 | 6.20 | 82.19 | -3.58 | 7.21 | 8.0 | 1030 | 37.8 |
| Example 23 | 1450 | 6.21 | 83.94 | -3.47 | 5.34 | 6.4 | 941 | 35.6 |
| Example 24 | 1350 | 6.21 | 80.93 | -3.32 | 7.72 | 8.4 | 1051 | 38.6 |
| Example 25 | 1400 | 6.21 | 80.82 | -3.35 | 7.00 | 7.8 | 948 | 38.9 |
| Example 26 | 1450 | 6.20 | 81.61 | -3.06 | 4.94 | 5.8 | 893 | 39.2 |

It could be confirmed that the sintered bodies stabilized with cerium and magnesium in Examples were sintered bodies having high aesthetic merit and a white to milky white color tone as well as high transmittance.

The entire contents of the description, the claims, the drawings and the abstract of Japanese Patent Application No. 2023-165892 filed September 27th, 2023 are hereby incorporated by reference as the disclosure of the description of the present disclosure.

**Claims**

1. A sintered body of zirconia, the sintered body comprising a color reducing agent and cerium as a stabilizing element, wherein the sintered body has a cerium content of 0.05 mol% or more and 15 mol% or less and a color reducing agent content of 0.01 mass% or more and less than 1.95 mass%.

2. The sintered body according to claim 1, comprising at least one selected from the group consisting of scandium, yttrium, praseodymium, gadolinium, terbium, erbium, magnesium and calcium as a stabilizing element other than cerium.

3. The sintered body according to claim 2, wherein a content of the stabilizing element other than cerium is more than 0 mol% and 2.8 mol% or less when the stabilizing element other than cerium is at least one selected from the group consisting of scandium, yttrium, praseodymium, gadolinium, terbium and erbium, and is more than 0 mol% and 9.8 mol% or less when the stabilizing element other than cerium is either or both of magnesium and calcium.

4. The sintered body according to any one of claims 1 to 3, comprising more than 0 mass% and 5.0 mass% or less of alumina.

5. The sintered body according to any one of claims 1 to 4, wherein the color reducing agent contains at least one element that has an 8-coordinate ionic radius larger than an ionic radius of a tetravalent cerium ion.

6. The sintered body according to any one of claims 1 to 5, wherein the color reducing agent contains at least one selected from the group consisting of lanthanum, neodymium, samarium, europium, dysprosium, holmium, thulium, ytterbium and lutetium.

7. The sintered body according to any one of claims 1 to 6, wherein the sintered body has a color tone (L*a*b*) satisfying formulae (1) and (2) below in L*a*b* color space:

Formula (1) …

$$0.34 \times L^* - 32.5 < a^* < 0.34 \times L^* - 20$$

Formula (2) …

$$-1.3 \times L^* + 70 < b^* < -1.3 \times L^* + 115.$$

8. The sintered body according to any one of claims 1 to 7, wherein the sintered body has a chroma C* of 12.0 or less.

9. The sintered body according to any one of claims 1 to 8, wherein the sintered body has a Vickers hardness Hv10 of 600 or more and 1200 or less.

10. The sintered body according to any one of claims 1 to 9, wherein the sintered body has a total luminous transmittance at a thickness of 1 mm of 20% or more and 70% or less.

11. A powder of zirconia, the powder comprising a color reducing agent source and a cerium compound as a stabilizing element source, wherein the powder has a cerium content of 0.05 mol% or more and 15 mol% or less and a color reducing agent content of 0.01 mass% or more and less than 1.95 mass%.

12. The powder according to claim 11, wherein the stabilizing element source includes the cerium compound and a compound of either or both of yttrium and magnesium.

13. The powder according to claim 11 or 12, wherein the color reducing agent source is a compound containing at least one selected from the group consisting of lanthanum, neodymium, samarium, europium, dysprosium, holmium, thulium, ytterbium and lutetium.

## INTERNATIONAL SEARCH REPORT

| International application No. |
| --- |
| **PCT/JP2024/034159** |

### A. CLASSIFICATION OF SUBJECT MATTER

*C04B 35/488*(2006.01)i
FI:   C04B35/488

According to International Patent Classification (IPC) or to both national classification and IPC

### B. FIELDS SEARCHED

Minimum documentation searched (classification system followed by classification symbols)

C04B35/488

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | WO 2021/117720 A1 (TOSOH CORP.) 17 June 2021 (2021-06-17) paragraphs [0174], [0176]-[0179], [0195]-[0200], [0204], [0211] | 1-6, 8-13 |
| A | | 7 |
| X | CN 103708831 A (YAAN YUANCHUANG CERAMIC CO., LTD.) 09 April 2014 (2014-04-09) paragraphs [0026]-[0027] | 1-13 |
| X | JP 1-033066 A (COUNCIL FOR SCIENTIFIC AND INDUSTRIAL RESEARCH) 02 February 1989 (1989-02-02) page 4, lower left column, line 16 to page 7, upper left column, line 6, tables I-III | 1, 5-6, 9, 11, 13 |
| X | JP 2017-132647 A (TOSOH CORP.) 03 August 2017 (2017-08-03) paragraphs [0057]-[0058], [0061] | 1-2, 5-6, 11-13 |
| X | JP 2012-211063 A (GC CORPORATION) 01 November 2012 (2012-11-01) paragraphs [0021]-[0024] | 1, 7-8 |

☑ Further documents are listed in the continuation of Box C.      ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **13 November 2024** | **26 November 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**

| International application No. |
| --- |
| **PCT/JP2024/034159** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2017-165599 A (KYOCERA CORPORATION) 21 September 2017 (2017-09-21)<br>entire text | 1-13 |
| A | JP 60-246261 A (TOYO SODA MFG CO., LTD.) 05 December 1985 (1985-12-05)<br>entire text, all drawings | 1-13 |
| A | JP 1-234365 A (SUMITOMO ELECTRIC INDUSTRIES, LTD.) 19 September 1989<br>(1989-09-19)<br>entire text, all drawings | 1-13 |

Form PCT/ISA/210 (second sheet) (July 2022)

## INTERNATIONAL SEARCH REPORT
### Information on patent family members

International application No.

**PCT/JP2024/034159**

| Patent document cited in search report | | | Publication date (day/month/year) | Patent family member(s) | Publication date (day/month/year) |
|---|---|---|---|---|---|
| WO | 2021/117720 | A1 | 17 June 2021 | US 2023/0014792 A1 paragraphs [0252]-[0258], [0273]-[0274], [0280]-[0281], tables 7, 9<br>EP 4074674 A1<br>CN 114787103 A<br>KR 10-2022-0110750 A | |
| CN | 103708831 | A | 09 April 2014 | (Family: none) | |
| JP | 1-033066 | A | 02 February 1989 | US 5017532 A column 4, line 38 to column 6, line 68, tables I-III<br>GB 2206111 A<br>DE 3821211 A1 | |
| JP | 2017-132647 | A | 03 August 2017 | (Family: none) | |
| JP | 2012-211063 | A | 01 November 2012 | US 2012/0252654 A1 paragraphs [0022]-[0024], table 1<br>EP 2505163 A1 | |
| JP | 2017-165599 | A | 21 September 2017 | (Family: none) | |
| JP | 60-246261 | A | 05 December 1985 | (Family: none) | |
| JP | 1-234365 | A | 19 September 1989 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021091602 A

- JP 2023165892 A

**Non-patent literature cited in the description**

- **SHANNON et al.** *Acta*, 1976, vol. A32, 751